# EUROPEAN PATENT SPECIFICATION

(11) **EP 3 025 708 B1**
(45) Date of publication and mention of the grant of the patent: **30.08.2017**
(21) Application number: 14828739.4
(22) Date of filing: 25.07.2014
(51) Int. Cl.: A61K 45/06, A61K 9/28, A61K 9/20, A61K 31/155, A61K 31/40, A61K 31/505

(54) **COMPLEX FORMULATION CONTAINING SUSTAINED RELEASE METFORMIN AND IMMEDIATE RELEASE HMG-COA REDUCTASE INHIBITOR**
KOMPLEXE FORMULIERUNG MIT VERZÖGERT FREIGESETZTEM METFORMIN UND UNMITTELBAR FREIGESETZTEM HMG-COA-REDUKTASE-HEMMER
FORMULATION COMPLEXE CONTENANT DE LA METFORMINE À LIBÉRATION PROLONGÉE ET UN INHIBITEUR DE LA HMG-COA RÉDUCTASE À LIBÉRATION IMMÉDIATE

(30) Priority: 25.07.2013 KR 20130088339
(43) Date of publication of application: 01.06.2016
(73) Proprietor: CJ Healthcare Corporation, Jung-gu, Seoul 100-400 (KR)
(72) Inventor: KANG, Kwi Man, Seoul 138-911 (KR); PARK, Young Joon, Seoul 137-040 (KR); PARK, Jun Hong, Yongin-si Gyeonggi-do 449-929 (KR); LEE, Ji Eun, Incheon 405-837 (KR); YOON, Seok Kee, Anyang-si Gyeonggi-do 431-908 (KR); KIM, Yu Jeong, Seoul 156-827 (KR); OH, Tack Oon, Hwaseong-si Gyeonggi-do 445-809 (KR); CHO, Tae Keun, Incheon 402-844 (KR)
(74) Representative: Sonn & Partner Patentanwälte
(86) International application number: PCT/KR2014/006797
(87) International publication number: WO 2015/012633

(56) References cited:
- EP-A1- 1 588 708
- WO-A1-03/105809
- WO-A2-2012/006398
- CN-A- 101 869 566
- KR-A- 20060 064 048
- KR-A- 20080 001 772
- KR-A- 20120 083 276
- KR-A- 20120 112 689
- KR-B1- 100 858 848
- US-A1- 2009 087 483

## Description

### Technical Field

The present invention relates to a combination formulation containing metformin used for treating non-insulin-dependent diabetes, etc., and an HMG-CoA reductase inhibitor used for treating dyslipidemia, and a preparation method thereof.

### Background Art

Diabetes is a chronic disease characterized by high blood glucose levels and can be divided into two types: type I diabetes, which causes the pancreas to stop producing insulin, and type II diabetes, which causes an increase of insulin resistance and functional deterioration of the pancreatic beta cells simultaneously.

In particular, type II diabetes, due to insulin resistance as well as high blood glucose levels, is a high-risk disease, which is very closely associated with cardiovascular diseases represented by health states, such as the states of obesity, hypertension, dyslipidemia, excessive blood coagulation, etc. Accordingly, it has been known that an appropriate control of an accompanying metabolic disease is necessary along with an active control of blood glucose levels, for its treatment.

Cardiovascular diseases are very common and serious diseases to diabetic patients. In fact, macrovascular complications such as coronary artery disease, cerebrovascular disease, and peripheral arterial disease account for about 75% of the causes of death of diabetic patients. Generally, diabetic patients have an about 2- to 4-fold higher risk of having cardiovascular disease than normal people. Since cardiovascular disease occurs at relatively young ages and spreads throughout the entire body, the death rate resulting from the disease is known to be very high. Furthermore, since the release of a research report disclosing that a diabetic patient with a history of cardiovascular disease can have the same level of occurrence of cardiovascular disease or a subsequent mortality risk thereof as that of a patient without diabetes but with a history of cardiovascular disease, the National Cholesterol Education Program (NCEP) Adult Treatment Panel III (ATP III) of the U.S. has regarded diabetes itself as a cardiovascular disease (CVD) risk equivalent, and has recommended that diabetic patients strictly follow the prevention guidelines at the same level as in the case of the patients with a history of cardiovascular disease.

Examples of therapeutic methods for reducing the risk of cardiovascular disease in diabetic patients may include the regulation of blood pressure, blood glucose levels, and lipid levels, and of these, the regulation of lipid is known to be most effective. According to domestic and foreign treatment guidelines, dyslipidemia of diabetic patients should be actively treated, and the use of an HMG-CoA reductase inhibitor, a statin-based drug, is recommended as an initial treatment. Accordingly, metformin, i.e., an insulin-independent treatment for diabetes due to the effect of lowering blood glucose levels, and an HMG-CoA reductase inhibitor, i.e., a treatment for dyslipidemia, are prescribed as the most effective methods for treating dyslipidemia in diabetic patients. However, when diabetic patients who simultaneously administer both the HMG-CoA reductase inhibitor and a hypoglycemic agent for oral administration were examined regarding their drug compliance for two years, the patients administering at least 80% of these drugs showed a drug compliance of 52% for the HMG-CoA reductase inhibitor and a drug compliance of 63% for the hypoglycemic agent for oral administration, thus confirming that the drug for treating dyslipidemia showed a relatively low drug compliance (*p*<0.001). Therefore, there is a need for the improvement in administration compliance thereof.

In this regard, the present invention provides a combination formulation containing metformin, a hypoglycemic agent for oral administration, and an HMG-CoA reductase inhibitor as active ingredients to improve the drug compliance described above.

Metformin is an effective hypoglycemic agent for oral administration widely used for prevention and treatment of occurrence and worsening of diabetes complications (e.g., cardiovascular disease, etc.). However, metformin is highly soluble in water and thus it is essential to prepare metformin in the form of a formulation capable of controlling its effective sustained-release, and this may cause a problem in developing a combination formulation containing metformin and other active ingredients having different properties. That is, due to the high water solubility of metformin, when metformin is formulated into a conventional tablet, it may be rapidly released to cause an excessive drop in blood glucose levels and may also cause gastrointestinal disorder. Additionally, metformin is conventionally administered in as much as 500 mg to 850 mg two or three times daily as a fast-release tablet (daily maximum of 2,550 mg), and thus the rapid change in blood glucose levels due to its fast release may provoke adverse reactions and resistance to metformin.

For the preparation of sustained-release metformin formulations, Korean Patent No. 10-0774774 discloses a method of controlling metformin release using a fatty acid ester derivative, which is a water-insoluble carrier for sustained-release, and International Publication No. WO 09/117130 discloses a method of controlling the release of water-soluble drugs containing metformin using a maximum 40% of waxes. However, in establishing sustained-release of the highly water-soluble drugs, if the drugs are impregnated into a polymer matrix using a water-insoluble agent or surrounded with a polymer membrane, there may occur a rapid release of the drugs at the initial stage due to the slow hydration rate of the polymer used therein, and the subsequent rapid change in its blood concentration may provoke adverse reactions and resistance to metformin due to the rapid change in the blood glucose levels. Additionally, it may have a drawback in that it requires a very large amount of the polymer for the establishment of sustained-release.

In order to solve these problems, International Publication Nos. WO 98/055107, WO 99/047125, WO 99/047128, WO 02/036100, and WO 03/028704, and Korean Patent Nos. 10-0772980, 10-0791844, and 10-1043816 disclose formulations of sustained-release metformin employing hydrophilic swellable polymers. In these sustained-release metformin formulations using hydrophilic swellable polymers, a stable drug-release pattern can be established by allowing an immediate hydration of a hydrophilic polymer in an aqueous solution. However, such swellable polymer adopted as a carrier for the sustained-release of metformin would cause a fatal problem in developing combination formulations, in that those agents having high viscosity and high molecular weight exist on the external surface of the granules and delay the release of drugs which are to be immediately released. Therefore, there is a need for the development of a technology to overcome this problem.

Meanwhile, the HMG-CoA reductase inhibitor has an excellent effect of lowering LDL-cholesterol level, can lower triglycerides level and increase HDL-cholesterol level while showing stability and drug-resistance with few adverse effects, and is thus widely used for the treatment of dyslipidemia. The HMG-CoA reductase inhibitor has a long half-life of 20 to 30 hours but its bioavailability is not high. Since it can be absorbed over the entire gastrointestinal tract, it is beneficial when the active ingredient can be rapidly released from a given formulation.

Reportedly, the HMG-CoA reductase inhibitor can be readily decomposed and/or oxidized when exposed to disadvantageous physical and/or chemical conditions. Therefore, numerous studies focused on the improvement of stability have long been carried out. For example, GB Patent Application Publication No. 2262229 discloses a pharmaceutical preparation of 7-substituted-3,5-dihydroxy-6-heptenoic acid sodium salt, which is an inhibitor of HMG-CoA reductase, and describes that the preparation requires an alkaline medium (e.g., carbonate, bicarbonate) capable of imparting a pH of at least 8 to an aqueous solution or dispersion of the composition.

Additionally, according to the previous report in a journal article (*determination of Rosuvastatin in the Pretence of Its Degradation Products by a Stability-Indicating LC Method* (journal of AOAC International Viol. 88, No. 4, 2005*)),* rosuvastatin calcium salt can be easily decomposed in an acidic condition of pH 5 or below, and by oxidation, sunlight, or high temperature, and as for a formulation, a granular product with a larger area exposed to the environment is less stable than an uncoated tablet, and a film-coated tablet is more stable than an uncoated tablet. Additionally, in another journal article (Stability study of cholesterol flowering statin drug in aqueous samples using HPLC and LC-MS (Environ Chem Lett (2010) 8; 185*)),* the degree of stability according to the pH of simvastatin, lovastatin, and pravastatin, and sunlight, or a solvent are disclosed.

In this regard, International Publication No. WO 00/35425 discloses an attempt to stabilize a statin mixture using a buffering agent capable of providing a pH of from 7 to 11, and Korean Patent No. 10-0388713 discloses a tribasic phosphate, Korean Patent No. 10-0698333 discloses a method of stabilizing rosuvastatin using a pharmaceutical composition, in which a counteranion is an inorganic salt instead of phosphate. Additionally, U.S. Patent Nos. 5686104 and 6126971 disclose that atorvastatin is stabilized by the addition of a pharmaceutically acceptable alkali earth metal.

In this aspect, for the manufacture of a combination formulation containing metformin and an HMG-CoA reductase inhibitor, it is necessary to properly control the *in vivo* release of each active ingredient while simultaneously ensuring that no adverse effects (e.g., deterioration in stability, etc.) may occur between the two ingredients due to the combination constitution of two different compounds. Specifically, the combination formulation should be designed in such a manner that a stable sustained-release pattern of the active ingredient be provided for metformin, whereas stability to decomposition and oxidation be provided for the HMG-CoA reductase inhibitor while simultaneously enabling an immediate-release pattern of the active ingredient.

However, as described above, the sustained-release agent for metformin may inhibit the immediate release of the HMG-CoA reductase inhibitor, thus making it difficult to design a formulation that can simultaneously meet the desired release rate for both ingredients. The stabilizing agent for the stability of the HMG-CoA reductase inhibitor may have a negative effect on the release rate, etc., of metformin. That is, there is a high risk that a sustained-release agent or a stabilizing agent, etc., which are contained in the combination formulation, may exert a negative effect on the mutual stability of the drugs and the release rates thereof, and thus it is not easy to design a combination formulation capable of securing optimum stability and release rate.

For the purpose of controlling the sustained-release of metformin, numerous studies have been performed on formulation types which can prevent a rapid release of drugs by constituting a matrix-type formulation containing a certain amount of swellable sustained-release agents with high molecular weight and high viscosity.

Generally, swellable agents for sustained-release are hydrophilic polymers having a three-dimensional network structure in which they are physically or chemically densely-bonded, and thus they become swollen and form a hydrated gel within a short period of time when they are brought into contact with an aqueous solution, thereby preventing the immediate release of drugs. However, when developing a combination formulation to simultaneously have both a gastric-retention type sustained-release using swellable agents and an immediate release for a drug to be released quickly at initial release, there is a problem in that the release of the drug for immediate release is delayed due to the high viscosity of the swellable agents for sustained-release.

In order to resolve these problems, International Application No. PCT/EP2003/004472 discloses a multi-layered tablet in which each drug is contained in a different layer and an inert layer is provided therebetween; and Korean Patent Application No. 10-2012-0120519 also discloses a multi-layered tablet, in which each drug is contained in a different layer and an intermediate layer which contains no drug is provided therebetween.

The technologies disclosed above attempted to minimize physical and chemical reactions between drugs using the multi-layered tablet system, thereby allowing improved stability and effective release of each drug. However, the multi-layered tablet has disadvantages in that the manufacture of the multi-layered tablets consisting of at least three layers causes a significant loss of ingredients during processing, increases the working hours, and also further increases the tablet mass due to the extra intermediate layer, thus making it difficult to apply such technology to a high dose metformin formulation for sustained-release.

Meanwhile, International Publication No. WO 03/026637 discloses a combination formulation manufactured by coating a water-insoluble polymer on a controlled release formulation as an intermediate layer while coating an immediate-release formulation on the outer layer, and Korean Patent No. 10-0705210 discloses a combination preparation in which a water-soluble polymer is coated on the sustained-release formulation as an intermediate layer while coating an immediate-release formulation on the outer layer. These technologies disclose coating an intermediate layer located in between two different drugs and thereby promote minimization of physical and chemical reactions, improve stability, and reduce the mass of tablets.

However, since the drug for immediate release should be coated on the top of a sustained-release formulation the crystal form of the active ingredients may be altered during the process of dissolving or dispersing the immediate release drug, and there is also a problem from the commercial aspect that it is difficult to secure content homogeneity through a coating process in large scale production. Additionally, it may be desirable to apply the technologies to those components which, as active ingredients, are contained in trace amounts in the formulation in the above-mentioned patents. However, there is a limitation to the application of these technologies to drugs with a relatively high dose, and thus it is not desirable to apply these technologies to an atorvastatin calcium salt formulation having a unit formulation dose of 10 mg to 80 mg.

Under these circumstances, the present inventors have made intensive efforts to develop a formulation capable of securing optimum stability and release rate, and as a result, have discovered that, by a combination formulation consisting of a first sustained-release composition which consists of granules containing metformin or a pharmaceutically acceptable salt thereof and a swellable polymer and a water-insoluble polymer film surrounding the granules, and a second immediate-release composition containing an HMG-CoA reductase inhibitor, a combination formulation with effectively improved stability can be provided by preventing physical and chemical reactions between active ingredients while simultaneously securing stable release of each active ingredient contained therein, thereby completing the present invention.

### Disclosure

### Technical Problem

The present invention aims to provide a combination formulation with effectively improved stability by preventing physical and chemical reactions between active ingredients while simultaneously securing stable release of each active ingredient contained therein, and a method of preparing the same.

### Technical Solution

An object of the present invention is to provide a pharmaceutical composition containing metformin, which treats non-insulin-dependent diabetes, etc., and a statin-based drug, which is used for treating dyslipidemia, etc. In detail, the present invention relates to a composition with a two-phase system, which is capable of controlling the initial burst release of a drug for sustained-release while rapidly releasing a drug for immediate release at the initial stage without being affected by a swellable agent with high viscosity and improving drug stability, via preparation of granules coated with a water-insoluble polymer, the granules containing metformin or a pharmaceutically acceptable salt thereof and a swellable polymer.

### Advantageous Effects of the Invention

The drug-release controlling system according to the present invention provides improved administration convenience and compliance by inhibiting initial burst release through double release control by using a swellable polymer and a water-insoluble polymer together, with even a small amount of polymer. Furthermore, the system of the present invention can control the delay in immediate-release by using a water-insoluble polymer film on sustained release granules. The drug-release controlling system according to the present invention thus provides an effective two-phase system of sustained-release and immediate-release.

### BRIEF DESCRIPTION OF THE DRAWINGS

FIG. 1 shows the results of a release test for the combination formulations prepared in Examples 1 and 2 of the present invention and a Glucophage XR^{®} 500 mg tablet.
FIG. 2 shows the results of a release test for the combination formulations prepared in Examples 1 and 2 of the present invention and a Lipitor 10 mg tablet.
FIG. 3 shows the results of a release test for the combination formulations prepared in Examples 3 and 4 of the present invention and a Crestor 10 mg tablet.

### BEST MODE

Preferred embodiments of the present invention will be described below in more detail with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art.

To solve the above problems, the present invention provides a combination formulation including a first sustained-release composition which includes granules containing metformin or a pharmaceutically acceptable salt thereof and a swellable polymer and a water-insoluble polymer film for coating the granules,
and a second immediate-release composition containing an HMG-CoA reductase inhibitor.

As used herein, the term "a first sustained-release composition" refers to a composition containing metformin or a pharmaceutically acceptable salt thereof capable of long-acting release of the same by preventing its rapid release. For sustained-release, metformin or a pharmaceutically acceptable salt thereof is formed into granules along with a swellable polymer, and each individual granule is coated with a water-insoluble polymer film.

As used herein, the term "metformin" refers to a compound with a chemical name of N,N-dimethylimidodicarbonimidic diamide (Formula 1 below), which is used as a therapeutic agent for preventing or treating non-insulin-dependent diabetes.

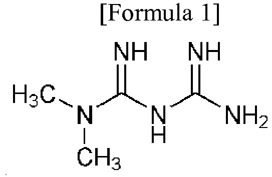

Metformin may be used by separation from natural resources, by manufacturing via chemical modification after obtainment thereof from natural resources, or easily by a chemical synthesis according to a known method by a skilled person in the art. Alternatively, commercially available metformin may be purchased for use.

Preferably, metformin or a pharmaceutically acceptable salt thereof may be contained in the combination formulation of the present invention in an amount from 250 mg to 1000 mg.

As used herein, the term "a swellable polymer" refers to a pharmaceutically acceptable polymer which becomes swollen in an aqueous solution, thereby allowing control of drug release. In the present invention, the swellable polymer forms granules along with metformin or a pharmaceutically acceptable salt thereof and exhibits the sustained-release characteristic of the same. The swellable polymer that can be used in the present invention may include at least one selected from the group consisting of hydroxypropyl methylcellulose, hydroxyethylcellulose, hydroxypropylcellulose, polyethylene oxide, carrageenan, natural gum, guar gum, tragacanth, acacia gum, locust bean gum, xanthan gum, polyvinyl alcohol, and polyvinylpyrrolidone, and preferably, hydroxypropyl methylcellulose or polyethylene oxide, but is not limited thereto as long as it is a pharmaceutically acceptable swellable polymer enabling controlled release according to the purposes of the present invention. Preferably, the swellable polymer has a viscosity of 100 cps or higher.

Preferably, the swellable polymer may be contained in the combination formulation in an amount from 10 wt% to 40 wt% based on the total weight of the first sustained-release composition. When the swellable polymer is contained at less than 10 wt%, it becomes difficult to achieve effective control of drug release, whereas when the swellable polymer is contained at more than 40 wt%, the size of tablets becomes too large to be administered, and is thus not appropriate.

The granules of the present invention containing metformin or a pharmaceutically acceptable salt thereof and the swellable polymer are formed by coating the external surface with a water-insoluble polymer.

As used herein, the term "a water-insoluble polymer" refers to a pharmaceutically acceptable polymer capable of controlling drug release, which is water-insoluble or hardly soluble in water. Additionally, the purposes of the water-insoluble polymer of the present invention not only include prevention of the release of metformin or a pharmaceutically acceptable salt thereof, but also prevention of the HMG-CoA reductase inhibitor contained in the second immediate-release composition from being in contact with the swellable polymer. That is, the combination formulation according to the present invention is formed such that the swellable polymer can be prevented from physical contact and chemical reaction with the HMG-CoA reductase inhibitor by the water-insoluble polymer film coating.

The present invention relates to a combination formulation which contains an HMG-CoA reductase inhibitor in addition to metformin, and the swellable polymer used for the sustained-release of metformin or a pharmaceutically acceptable salt thereof inhibits the release of the HMG-CoA reductase inhibitor, and also increases the amount of impurities of the HMG-CoA reductase inhibitor, thereby significantly deteriorating the stability of the formulation. Accordingly, the type of the formulation, which contains a swellable polymer for sustained-release metformin but does not affect the HMG-CoA reductase inhibitor, should be considered. For this purpose, in the present invention, the external surface of the granules, which contain metformin or pharmaceutically acceptable salt and a swellable polymer, is coated with a water-insoluble polymer, thereby preventing the contact between the swellable polymer and the HMG-CoA reductase inhibitor.

In an exemplary embodiment of the present invention, when the water-insoluble polymer was absent, the swellable polymer exhibited an impact on the HMG-CoA reductase inhibitor, thereby reducing the release rate of the HMG-CoA reductase inhibitor and increasing the formation of impurities. On the contrary, in the case of a combination formulation of the present invention using a water-insoluble polymer film, the first sustained-release composition and the second immediate-release composition of respectively showed a release pattern similar to that of a single-formulation type, and there was no increase in the formation of impurities. Since the first sustained-release composition according to the present invention did not cause a high viscosity problem by the swellable polymer while effectively controlling the sustained-release metformin, these results suggest that the sustained-release agent for metformin did not affect the immediate release of the second immediate-release composition.

The water-insoluble polymer that can be used in the present invention may include at least one selected from the group consisting of methacrylic acid copolymer, ethylcellulose, hydroxypropyl methylcellulose phthalate, hydroxypropyl methylcellulose acetate succinate, cellulose acetate phthalate, fatty acids, fatty acid esters, fatty acid alcohols, and waxes, and preferably, methacrylic acid copolymer or ethylcellulose, but the water-insoluble polymer is not limited thereto as long as it is a pharmaceutically acceptable water-insoluble polymer enabling controlled release according to the purposes of the present invention.

Preferably, the water-insoluble polymer according to the present invention may be contained in the amount from 1 wt% to 20 wt% based on the total weight of the first sustained-release composition. When the polymer content exceeds 20 wt%, it will increase the film thickness and slow hydration of the swellable polymer, and thus is not suitable for controlling the initial release of drug.

As used herein, the term "a second immediate-release composition" refers to a composition containing an HMG-CoA reductase inhibitor which can be completely disintegrated in distilled water at 37°C within 5 minutes.

As used herein, the term "HMG-CoA," as an acronym for "3-hydroxy-3-methylglutaryl-coenzyme A", refers to a precursor for biosynthesis of sterols including cholesterols. As used herein, the term "HMG-CoA reductase inhibitor" refers to compounds which provide the effect of lowering the levels of *in vivo* total cholesterol and LDL-cholesterol by inhibiting the activity of HMG-CoA reductase, which is involved in the early stages of the conversion of HMG-CoA into mevalonate during the process of cholesterol biosynthesis. For example, the HMG-CoA reductase inhibitor may be at least one selected from rosuvastatin, atorvastatin, pitavastatin, lovastatin, simvastatin, pravastatin, and fluvastatin, or a pharmaceutically acceptable salt, but it is not limited thereto. Additionally, the formulation of the present invention may further include a pharmaceutically acceptable alkalifying agent. The HMG-CoA reductase inhibitor according to the present invention is preferably atorvastatin or rosuvastatin. Preferably, the HMG-CoA reductase inhibitor may be contained in the combination formulation of the present invention in an amount from 5 mg to 160 mg. Additionally, in the case of rosuvastatin, it may be contained in the combination formulation of the present invention in an amount from 5 mg to 40 mg.

The second immediate-release composition may further contain a pharmaceutically acceptable disintegrant and/or a dissolution adjuvant for the purpose of being completely disintegrated in distilled water at 37°C within 5 minutes. The disintegrant and/or the dissolution adjuvant that can be used in the present invention may include at least one selected from the group consisting of croscarmellose sodium, sodium starch glycolate, crospovidone, sodium carboxymethyl cellulose, low-substituted hydroxypropyl cellulose, polysorbate, poloxamer, and sodium lauryl sulfate, and preferably, croscarmellose sodium or crospovidone may be used, but the disintegrant and/or the dissolution adjuvant are not limited thereto as long as they are pharmaceutically acceptable additives capable of controlling the disintegration according to the purposes of the present invention.

The combination pharmaceutical formulation according to the present invention can maintain stability without any changes in characteristics even when the two drugs of metformin or a pharmaceutically acceptable salt thereof and an HMG-CoA reductase inhibitor are manufactured and stored in a combination process.

As used herein, the term "a pharmaceutically acceptable salt" refers to a formulation type that does not damage the biological activities and physical properties of metformin or an HMG-CoA reductase inhibitor to be administered. The pharmaceutically acceptable salt may include acid addition salts which can form non-toxic acid addition salts containing pharmaceutically acceptable anions, e.g., inorganic acids such as hydrochloric acid, sulfuric acid, nitric acid, phosphoric acid, hydrobromic acid, and hydriodic acid; organic carbonic acids such as tartaric acid, formic acid, citric acid, acetic acid, trichloroacetic acid, trifluoroacetic acid, gluconic acid, benzoic acid, lactic acid, fumaric acid, maleic acid, and salicylic acid; sulfonic acids such as methanesulfonic acid, ethanesulfonic acid, benzenesulfonic acid, and *p*-toluenesulfonic acid; etc. For example, the pharmaceutically acceptable salt may include metal salts or alkali earth metal salts formed by lithium, sodium, potassium, calcium, magnesium, etc.; amino acid salts such as lysine, arginine, guanidine, etc.; organic salts such as dicyclohexylamine, N-methyl-D-glucamine, tris(hydroxymethyl)methylamine, diethanolamine, choline, and triethylamine; etc.

Additionally, the combination pharmaceutical formulation of the present invention may further include a film layer on the external surface. The film layer may be, for example, a shield film layer, a moisture-proofing film layer, or a glucose film layer, etc. Preferably, the external film layer is formed of a water-soluble material, which may include hydroxypropyl methylcellulose, hydroxypropylcellulose, hydroxyethylcellulose, cellulose acetate phthalate, ethylcellulose, methylcellulose, polymethacrylate, polyvinyl alcohol-polyethylene glycol graft copolymer (Kollicoat^{®}; BASF, Germany), polyvinyl alcohol (Opadry^{®}; Colorcon, USA), or a combination thereof, but are not limited thereto.

Additionally, the combination pharmaceutical formulation of the present invention may be formulated by further using additives conventionally used in the art within the scope of not damaging the effects of the present invention, such as a diluent, a binder, a lubricant, a pH adjuster, an antifoaming agent, a dissolution adjuvant, an antioxidant, etc.

The combination pharmaceutical formulation of the present invention may be prepared in various formulation types, e.g., tablets such as uncoated tablets, film-coated tablets, single-layer tablets, double-layer tablets, multi-layer tablets, or core tablets; powders; granules; capsules; etc. Preferably, the combination pharmaceutical formulation of the present invention is prepared in the form of a double-layer tablet consisting of the first sustained-release composition and the second immediate-release composition.

The thus-prepared combination pharmaceutical formulation of the present invention can provide appropriate release features suitable for each of the pharmaceutical active ingredients by continuously releasing metformin and rapidly releasing HMG-CoA reductase inhibitor during *in vivo* administration. Additionally, administration convenience was improved by reducing the contents of sustained-release agents necessary for sustained-release metformin, whereas the drug for immediate-release was rapidly dissolved and the stability of the HMG-CoA reductase inhibitor was improved by including a stabilizing agent. Accordingly, the combination pharmaceutical formulation of the present invention can be effectively used for the prevention and treatment of dyslipidemia, dyslipidemia, atherosclerosis, diabetes, and diabetes complications.

In another exemplary embodiment, the present invention provides a method of preparing the combination formulation including preparing the first sustained-release composition, by preparing granules containing metformin or a pharmaceutically acceptable salt thereof, and a swellable polymer, followed by forming a water-insoluble polymer film on the granules;
preparing the second immediate-release composition containing an HMG-CoA reductase inhibitor; and
formulating the first sustained-release composition and the second immediate-release composition into a unit formulation.

Additionally, the method of the present invention may further include forming a film layer on the external surface of the combination formulation.

As used herein, the terms "first sustained-release composition", "metformin", "swellable polymer", "water-insoluble polymer", "a second immediate-release composition", "HMG-CoA", "a pharmaceutically acceptable salt", and "film layer" are the same as described above.

### DETAILED DESCRIPTION OF THE INVENTION

Hereinafter, the present invention will be described in more detail with reference to the following Examples. However, these Examples are for illustrative purposes only, and the invention is not intended to be limited by these Examples.

### Example 1

### 1) Preparation of sustained-release metformin hydrochloride granules

Sustained-release metformin hydrochloride granules were prepared according to the composition and the content shown in Table 1. Specifically, metformin hydrochloride and colloidal silicon dioxide were passed through a 20-mesh sieve and mixed with polyethylene oxide (Polyox^{®} WSR301). Then, the resultant was sprayed with a binder solution in which a solvent mixture containing isopropyl alcohol, acetone, and purified water mixed in a ratio of 6:3:1 was dissolved at a concentration of 10 w/v% with addition of a methacrylic acid copolymer (Eudragit^{®} RS PO), then dried in a fluidized bed granulator for granulation, and the resultant was passed through a 20-mesh sieve. The thus-obtained granulated product was treated with magnesium stearate and mixed to prepare sustained-release metformin hydrochloride granules.

**Table 1**

| | | |
|---|---|---|
| Active Ingredient | metformin hydrochloride | 500 mg |
| Lubricant | colloidal silicon dioxide | 5 mg |
| Water-insoluble Agent | methacrylic acid copolymer (Eudragit^{®} RS PO) | 50 mg |
| Swelling agent | polyethylene oxide (Polyox^{®} WSR301) | 150 mg |
| Lubricant | magnesium stearate | 5 mg |
| Total Weight | | 710 mg |

### 2) Preparation of immediate-release atorvastatin granules

Immediate-release atorvastatin granules were prepared according to the composition and the content shown in Table 2. Specifically, atorvastatin calcium salt, precipitated calcium carbonate, microcrystalline cellulose, lactose hydrate, and croscarmellose sodium were mixed, treated with hydroxypropylcellulose dissolved in 20% ethanol to obtain granules, and the resulting granules were dried in a fluidized bed dryer and then passed through a 20-mesh sieve. The thus-obtained granulated product was treated with croscarmellose sodium and magnesium stearate, and mixed to prepare immediate-release atorvastatin granules.

**Table 2**

| | | |
|---|---|---|
| Active Ingredient | Atorvastatin Calcium Salt | 10.85 mg |
| Diluent | precipitated calcium carbonate | 20 mg |
| Diluent | microcrystalline cellulose | 58 mg |
| Diluent | lactose hydrate | 42.8 mg |
| Surfactant | Polysorbate 80 | 0.6 mg |
| Binder | hydroxypropylcellulose | 3 mg |
| Disintegrant | croscarmellose sodium | 14 mg |
| Lubricant | magnesium stearate | 0.75 mg |
| Total Weight | | 150 mg |

### 3) Tableting of double-layer tablets

The metformin hydrochloride and atorvastatin granules prepared in 1) and 2) above were tableted into double-layer tablets in the amounts of 710 mg and 150 mg, respectively, thereby preparing white tablets having a unit weight of 860 mg per tablet.

### 4) Preparation of coating solution and coating

A coating pan (Hi-coater, Freund) was filled with the tablets prepared in 3) above and the exhaust air temperature was maintained at about 30°C to 40°C. 10 g of an Opadry^{®} 03B64650 (62.5% hydroxypropyl methylcellulose 2910, 30.79% titanium oxide, 6.25% polyethylene glycol 400, 0.27% yellow iron oxide, 0.18% red iron oxide, and 0.01% indigo carmine aluminum lake) coating agent was dissolved in 90 g of water to prepare a coating solution, which was sprayed on dried tablets using a sprayer operated under air pressure, and dried by further providing inlet air flow for about 10 minutes, thereby affording 885 mg of unit tablets of the present invention, in which the amount of coating per tablet was 25 mg.

### Example 2

Suspended-release metformin hydrochloride granules were prepared according to the composition and the content shown in Table 3. Specifically, metformin hydrochloride and colloidal silicon dioxide were passed through a 20-mesh sieve and mixed with hypromellose (Metolose^{®} 90SH-100,000 cps). Then, the resultant was sprayed with a binder solution in which a solvent mixture containing isopropyl alcohol, acetone, and purified water mixed in a ratio of 6:3:1 was dissolved at a concentration of 10 w/v% with addition of a methacrylic acid copolymer (Eudragit^{®} RS PO), then dried in a fluidized bed granulator for granulation, and the resultant was passed through a 20-mesh sieve. The thus-obtained granulated product was mixed with magnesium stearate to prepare final sustained-release metformin hydrochloride granules. The processes of preparing immediate-release atorvastatin granules, tableting of double-layer tablets, and coating were performed in the same manner as in Example 1.

**Table 3**

| | | |
|---|---|---|
| Active Ingredient | metformin hydrochloride | 500 mg |
| Lubricant | colloidal silicon dioxide | 5 mg |
| Water-insoluble Agent | methacrylic acid copolymer (Eudragit^{®} RS PO) | 50 mg |
| Swelling Agent | hypromellose (Metolose^{®} 90SH-100,000cps) | 150 mg |
| Lubricant | magnesium stearate | 5 mg |
| Total Weight | | 710 mg |

### Example 3

Immediate-release rosuvastatin granules were prepared according to the composition and the content shown in Table 4. Specifically, anhydrous calcium hydrogen phosphate was used as a stabilizing agent, and specifically, rosuvastatin calcium salt, microcrystalline cellulose, lactose hydrate, crospovidone, and magnesium stearate were mixed with anhydrous calcium hydrogen phosphate to prepare immediate-release rosuvastatin granules. The processes of preparing suspended-release metformin granules, tableting of double-layer tablets, and coating were performed in the same manner as in Example 1.

**Table 4**

| | | |
|---|---|---|
| Active Ingredient | rosuvastatin calcium salt | 10.4 mg |
| Diluent | anhydrous calcium hydrogen phosphate | 20.9 mg |
| Diluent | microcrystalline cellulose | 30.3 mg |
| Diluent | lactose hydrate | 78.4 mg |
| Disintegrant | crospovidone | 8.0 mg |
| Lubricant | magnesium stearate | 2.0 mg |
| Total Weight | | 150 mg |

### Example 4

Immediate-release rosuvastatin granules were prepared in the same manner as in Example 3, and the processes of preparing suspended-release metformin granules, tableting of double-layer tablets, and coating were performed in the same manner as in Example 2.

### Comparative Example 1

Immediate-release atorvastatin granules were prepared in the same manner as in Example 1 and then tableted into single tablets. The amount of coating per tablet was 5 mg, and 155 mg of unit tablets were obtained therefrom.

### Comparative Example 2

Immediate-release rosuvastatin granules were prepared in the same manner as in Example 3 and then tableted into single tablets. The amount of coating per tablet was 5 mg, and 155 mg of unit tablets were obtained therefrom.

### Comparative Examples 3 and 4

Suspended-release metformin hydrochloride granules were prepared according to the composition and the content shown in Table 5. Specifically, metformin hydrochloride and colloidal silicon dioxide were passed through a 20-mesh sieve, mixed with microcrystalline cellulose, and treated with polyvinylpyrrolidone (K-30) dissolved in distilled water for granulation. The resultant was dried in a fluidized bed dryer and passed through a 20-mesh sieve. The thus-obtained granulated product was mixed with polyethylene oxide (Polyox^{®} WSR301) and magnesium stearate, and mixed to prepare final sustained-release metformin hydrochloride granules.

**Table 5**

| | | |
|---|---|---|
| Active Ingredient | metformin hydrochloride | 500 mg |
| Lubricant | colloidal silicon dioxide | 5 mg |
| Excipient | microcrystalline cellulose | 50 mg |
| Binder | polyvinylpyrrolidone (K-30) | 10 mg |
| Swelling Agent | polyethylene oxide (Polyox^{®} WSR301) | 200 mg |
| Lubricant | magnesium stearate | 5 mg |
| Total Weight | | 770 mg |

The processes of preparing immediate-release atorvastatin granules, tableting of double-layer tablets, and coating were performed in the same manner as in Example 1, and the amount of the tablets finally obtained was 945 mg (Comparative Example 3).

The processes of preparing immediate-release rosuvastatin granules, tableting of double-layer tablets, and coating were performed in the same manner as in Example 3, and the amount of the tablets finally obtained was 945 mg (Comparative Example 4).

### Comparative Examples 5 and 6

Suspended-release metformin hydrochloride granules were prepared according to the composition and the content shown in Table 6. Specifically, metformin hydrochloride and colloidal silicon dioxide were passed through a 20-mesh sieve, mixed with microcrystalline cellulose, and treated with polyvinylpyrrolidone (K-30) dissolved in distilled water for granulation. The resultant was dried in a fluidized bed dryer and passed through a 20-mesh sieve. The thus-obtained granulated product was mixed with hypromellose (Metolose^{®} 90SH-100,000 cps) and magnesium stearate, and mixed to prepare final sustained-release metformin hydrochloride granules.

**[Table 6]**

| | | |
|---|---|---|
| Active Ingredient | metformin hydrochloride | 500 mg |
| Lubricant | colloidal silicon dioxide | 5 mg |
| Excipient | microcrystalline cellulose | 50 mg |
| Binder | polyvinylpyrrolidone (K-30) | 10 mg |
| Swelling Agent | hypromellose (Metolose^{®} 90SH-100,000cps) | 200 mg |
| Lubricant | magnesium stearate | 5 mg |
| Total Weight | | 770 mg |

The processes of preparing immediate-release atorvastatin granules, tableting of double-layer tablets, and coating were performed in the same manner as in Example 1, and the amount of the tablets finally obtained was 945 mg (Comparative Example 5).

The processes of preparing immediate-release rosuvastatin granules, tableting of double-layer tablets, and coating were performed in the same manner as in Example 3, and the amount of the tablets finally obtained was 945 mg (Comparative Example 6).

### Examples 5 to 8

Sustained-release metformin hydrochloride granules were prepared according to the composition and the content shown in Table 7. Specifically, sustained-release metformin hydrochloride granules were prepared in the same manner as in Example 1, except that they were prepared using the ingredients of Eudragit^{®} S100, Ethocel^{®} Std 14, cetyl alcohol, and Kollicoat^{®} SR 30D, respectively (See Table 7), instead of a methacrylic acid copolymer (Eudragit^{®} RS PO). The amount of each tablet obtained after tableting into double-layer tablets and completing the coating process was 885 mg.

**Table 7**

| | Example 5 | Example 6 | Example 7 | Example 8 |
|---|---|---|---|---|
| Metformin hydrochloride | 500 mg | 500 mg | 500 mg | 500 mg |
| Colloidal silicon dioxide | 5 mg | 5 mg | 5 mg | 5 mg |
| Polyethylene oxide (Polyox^{®} WSR301) | 150 mg | 150 mg | 150 mg | 150 mg |
| Methacrylic acid copolymer (Eudragit^{®} S100) | 50 mg | - | - | - |
| Ethylcellulose (Ethocel^{®} Std 14) | - | 50 mg | - | - |
| Waxes (cetyl alcohol) | - | - | 50 mg | - |
| Polyvinyl acetate (Kollicoat^{®} SR 30D) | - | - | - | 50 mg |
| Magnesium stearate | 5 mg | 5 mg | 5 mg | 5 mg |
| Total | 710 mg | 710 mg | 710 mg | 710 mg |

### Experimental Example 1: Release test of metformin

In order to confirm whether the combination formulation according to the present invention can exhibit a release rate equivalent to that of a Glucophage XR^{®} 500 mg tablet, a reference drug, a release test was performed for the combination formulations prepared above.

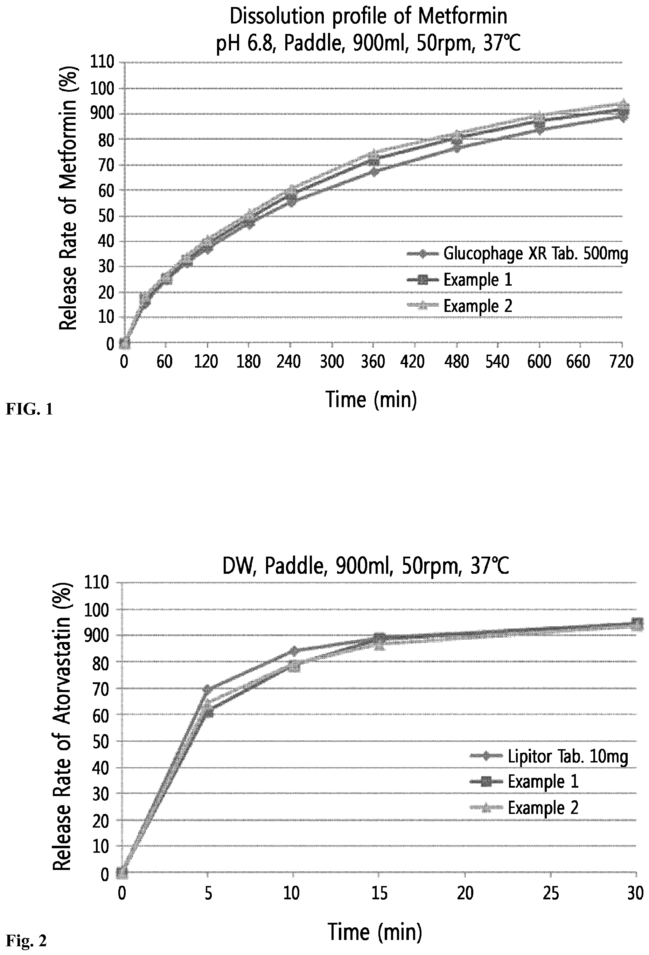

Specifically, the sustained-release formulations prepared in Examples 1 and 2 and Comparative Examples 3 and 5, and the Glucophage XR^{®} 500 mg tablet as in commercial sale, which was used as a reference, were tested at 37°C in 900 mL of dissolution media of phosphate buffer (pH 6.8) at 50 rpm according to the dissolution method (Method II) in USP. The samples were collected at scheduled times and analyzed via HPLC to calculate the release rates. The results are shown in Table 8 and FIG. 1.

The conditions used for HPLC are as follows.
Column: Waters XBridge (C18, 150 mm × 4.6 mm, 5 µm)
Detector: spectrophotometric detector (218 nm)
Mobile phase: solution prepared by dissolving 17 g of NH₄H₂PO₄ in 1 L of water and adjusting pH thereof to 3.0 with phosphoric acid
Flow rate: 1.0 mL/min
Column temperature: 40°C
Time of analysis: 4 min

**[Table 8]**

| Release Time (min) | Release Rate of Metformin Hydrochloride (%) | | | | |
|---|---|---|---|---|---|
| | Example 1 | Example 2 | Comparative Example 3 | Comparative Example 5 | Glucophage XR^{®} 500 mg Tablet |
| 30 | 17.3±1.6 | 18.6±0.1 | 16.2±0.1 | 17.6±0.5 | 15.8±3.2 |
| 60 | 25.3±1.3 | 26.4±1.1 | 25.7±0.2 | 26.5±0.5 | 24.9±3.1 |
| 90 | 32.4±2.2 | 34.1±1.2 | 33.6±0.5 | 34.8±0.7 | 31.6±3.2 |
| 120 | 39.0±2.4 | 40.7±1.5 | 40.1±0.3 | 41.1±0.9 | 37.1±3.4 |
| 180 | 48.8±3.4 | 50.8±2.0 | 51.6±0.7 | 52.0±1.2 | 47.1±2.8 |
| 240 | 58.4±3.9 | 60.7±2.0 | 60.9±1.0 | 61.2±1.5 | 55.2±3.0 |
| 360 | 72.1±4.6 | 74.6±1.9 | 75.4±1.0 | 76.1±1.8 | 67.3±3.1 |
| 480 | 80.4±3.9 | 82.4±1.4 | 85.6±0.7 | 86.3±1.6 | 76.8±2.4 |
| 600 | 87.2±2.1 | 89.4±0.7 | 90.7±0.1 | 92.1±1.4 | 83.9±2.3 |
| 720 | 92.0±1.3 | 94.2±0.1 | 94.6±0.1 | 96.7±0.6 | 88.9±2.3 |

The release tests were performed for metformin hydrochloride on the combination formulations prepared in Examples 1 and 2 and Comparative Examples 3 and 5, and the results were compared with that of the Glucophage XR^{®} 500 mg tablet, the reference drug. The results show that the combination formulations exhibited release rates similar to that of the Glucophage XR^{®} 500 mg tablet. From the results, it was confirmed the drug release was effectively controlled using the swellable polymer and the water-insoluble polymer. The release control is highly significant considering that the tablet mass of the Glucophage XR^{®} 500 mg tabletis 1,000 mg or higher whereas the tablet mass of combination formulations according to the present invention is 900 mg or less.

Additionally, the sustained-release tablets prepared in Examples 5, 6, 7, and 8 according to the kinds of water-insoluble polymers, and the commercial product, the Glucophage XR^{®} 500 mg tablet, which was used as a control drug, were analyzed under the same conditions as described above, and the results are shown in Table 9 below. As a result of the analysis, the sustained-release tablets prepared in Examples 5, 6, 7, and 8 were shown to have a release pattern similar to that of the Glucophage XR^{®} 500 mg tablet, a control drug. From this result, it was confirmed that the drug release was effectively controlled using the swellable polymer and the water-insoluble polymer.

**Table 9**

| Release Time (min) | Release Rate of Metformin Hydrochloride (%) | | | | |
|---|---|---|---|---|---|
| | Example 5 | Example 6 | Example 7 | Example 8 | Glucophage XR^{®} 500 mg Tablet |
| 30 | 21.2±2.9 | 23.8±3.1 | 24.4±2.8 | 23.9±2.9 | 15.8±3.2 |
| 60 | 28.0±2.1 | 30.6±2.8 | 33.4±2.6 | 33.2±1.8 | 24.9±3.1 |
| 90 | 36.0±1.5 | 41.7±2.1 | 43.8±2.5 | 42.4±1.8 | 31.6±3.2 |
| 120 | 42.7±1.2 | 50.0±1.9 | 52.9±2.1 | 49.8±1.0 | 37.1±3.4 |
| 180 | 53.8±1.1 | 63.2±1.8 | 66.9±2.0 | 61.0±1.1 | 47.1±2.8 |
| 240 | 65.1±0.9 | 73.9±1.9 | 76.4±2.1 | 69.5±0.9 | 55.2±3.0 |
| 360 | 78.9±0.9 | 85.4±1.5 | 89.0±1.8 | 81.9±0.8 | 67.3±3.1 |
| 480 | 87.2±0.6 | 91.4±1.3 | 92.2±1.2 | 87.8±0.8 | 76.8±2.4 |
| 600 | 92.3±0.5 | 92.6±0.9 | 99.4±0.8 | 92.7±0.5 | 83.9±2.3 |
| 720 | 94.4±0.4 | 97.1±0.8 | 99.5±0.8 | 93.6±0.2 | 88.9±2.3 |

### Experimental Example 2: Release test of atorvastatin

In order to confirm whether the combination formulations according to the present invention can constantly maintain concentration in the blood via immediate release of a fast-release drug, a Lipitor^{®} 10 mg tablet, i.e., a control drug of atorvastatin calcium salt, the formulations prepared in Comparative Examples 1, 3, and 5, and Examples 1 and 2 were tested at 37°C in 900 mL of dissolution media of distilled water at 50 rpm according to the dissolution method (Method II) in USP. The samples were collected at scheduled times and analyzed via HPLC to calculate the release rates. The results are shown in Table 10 and FIG. 2.

The conditions used for HPLC are as follows.
Column: Phenomenex Luna (C18, 250 mm × 4.6 mm, 5 µm)
Detector: spectrophotometric detector (244 nm)
Mobile phase: 0.05 mol/L ammonium citrate (pH 4.0):ACN:THF = 2:2:1
Flow rate: 1.5 mL/min
Column temperature: 40°C
Time of analysis: 4 min

**Table 10**

| Time for Release (min) | Release Rate of Atorvastatin (%) | | | | | |
|---|---|---|---|---|---|---|
| | Example 1 | Example 2 | Comparative Example 1 | Comparative Example 3 | Comparative Example 5 | Lipitor^{®} 10 mg Tablet |
| 5 | 61.2±2.5 | 64.5±3.0 | 65.1±5.3 | 41.3±21.7 | 42.5±10.9 | 69.5±2.5 |
| 10 | 78.8±3.1 | 79.1±2.0 | 86.9±3.3 | 54.1±14.0 | 62.7±7.7 | 84.4±1.5 |
| 15 | 88.7±2.8 | 86.8±2.3 | 93.6±1.3 | 53.6±12.9 | 72.2±5.4 | 89.1±1.6 |
| 30 | 94.6±1.8 | 93.8±2.1 | 97.5±0.6 | 78.6±9.0 | 81.6±4.2 | 94.6±1.6 |
| Disintegration Time | 3 min 20 sec | 3 min 10 sec | 3 min 10 sec | 10 min 50 sec | 9 min 40 sec | 3 min 10 sec |

In the above experiment, the formulation of Comparative Example 1 (atorvastatin single tablets) showing a release rate similar to that of the Lipitor^{®} 10 mg tablet, a control drug, was prepared and its release rate was evaluated. When combination formulations were prepared using atorvastatin granules, which were prepared in the same manner as in Comparative Example 1, according to the methods in Comparative Example 3 and Comparative Example 5, there occurred a delay in disintegration of the immediate-release layer of atorvastatin granules due to a swellable polymer, thus lowering the release rate.

Meanwhile, in Examples 1 and 2, in which sustained-release metformin granules were prepared by coating a water-insoluble polymer on a swellable polymer and metformin hydrochloride, the disintegration time of atorvastatin was secured at a level the same as or similar to that of the atorvastatin single tablets. That is, it was confirmed that, by preventing the physical contact between two granules according to the present invention, the immediate-release atorvastatin hydrochloride granules were disintegrated and released in a similar manner to that of the single tablets, and this suggests that the same can be effectively applied to a combination formulation requiring a two-phase system consisting of a sustained-release formulation and a fast-release formulation requiring immediate release.

Additionally, the same experiment was performed for the formulations prepared in Examples 5 to 8. The formulations of Examples 5 to 8 were prepared by coating a different water-insoluble polymer on a swellable polymer and metformin hydrochloride, instead of a methacrylic acid copolymer (Eudragit^{®} RS PO), in which the atorvastatin granules prepared in the same manner as in Comparative Example 1 were tableted into double-layer tablets. The results of the release test are shown in Table 11 below.

**Table 11**

| Time for Release (min) | Release Rate of Atorvastatin (%) | | | | |
|---|---|---|---|---|---|
| | Example 5 | Example 6 | Example 7 | Example 8 | Lipitor^{®} 10 mg Tablet |
| 5 | 62.1±3.5 | 61.7±2.7 | 61.9±3.1 | 63.4±2.8 | 69.5±2.5 |
| 10 | 79.4±2.9 | 78.4±2.5 | 79.5±2.7 | 79.8±1.9 | 84.4±1.5 |
| 15 | 89.1±1.9 | 90.1±2.9 | 90.4±3.1 | 91.4±1.4 | 89.1±1.6 |
| 30 | 95.1±3.1 | 94.8±2.1 | 93.4±1.8 | 93.9±1.1 | 94.6±1.6 |
| Disintegration Time | 4 min 30 sec | 4 min 50 sec | 4 min 40 sec | 4 min 10 sec | 3 min 10 sec |

The release rate of atorvastatin was shown to be similar to that of the formulation of Comparative Example 1, without being affected by the swellable polymer with high viscosity. Additionally, when a water-insoluble polymer other than the methacrylic acid copolymer (Eudragit^{®} RS PO) was used, the effect of preventing the physical contact between two granules was shown to be identical.

### Experimental Example 3: Release test of rosuvastatin

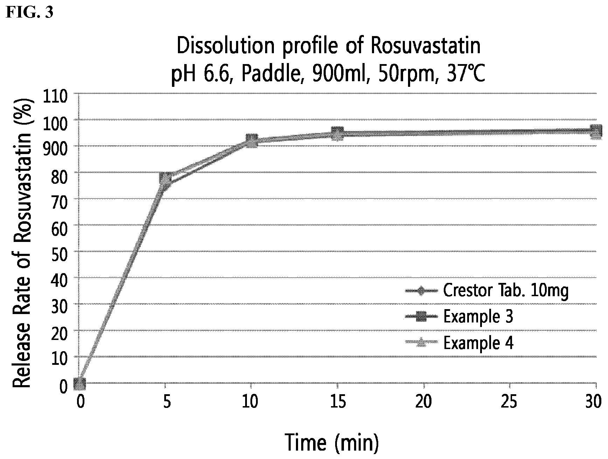

In order to confirm the release rate for the rosuvastatin calcium salt formulation, a Crestor^{®} 10 mg tablet, i.e., a control drug of rosuvastatin calcium salt, and the formulations prepared in Comparative Examples 2, 4, and 6 and Examples 3 and 4 were tested at 37°C in 900 mL of a citrate buffer solution (pH 6.6) at 50 rpm according to the resolution method (Method II) in USP. The samples were collected at scheduled times and analyzed via HPLC to calculate the release rates. The results are shown in Table 12 and FIG. 3.

The conditions used for HPLC are as follows.
Column: Capcell Pak (C18, 75 mm × 4.6mm, 3 µm)
Detector: spectrophotometric detector (242 nm)
Mobile phase: purified water:ACN:phosphoric acid = 600:400:1
Flow rate: 1.0 mL/min
Column temperature: room temperature
Time of analysis: 5 min

**Table 12**

| Time for Release (min) | Release Rate of Rosuvastatin (%) | | | | | |
|---|---|---|---|---|---|---|
| | Example 3 | Example 4 | Comparative Example 2 | Comparative Example 4 | Comparative Example 6 | Crestor^{®} 10 mg Tablet |
| 5 | 78.3±5.1 | 78.1±3.7 | 76.8±4.8 | 58.4±7.1 | 63.4±8.1 | 75.3±7.0 |
| 10 | 92.4±3.8 | 91.9±2.3 | 91.4±2.7 | 84.1±6.1 | 86.1±3.7 | 91.6±3.4 |
| 15 | 95.2±2.2 | 94.7±0.9 | 94.4±1.1 | 87.4±3.1 | 88.4±3.4 | 94.2±1.8 |
| 30 | 96.1±1.7 | 95.1±0.8 | 95.1±0.8 | 89.1±2.7 | 89.4±3.2 | 95.6±1.7 |
| Disintegr ation Time | 2 min 40 sec | 2 min 40 sec | 2 min 50 sec | 9 min 30 sec | 8 min 50 sec | 2 min 50 sec |

In the above experiment, the formulation of Comparative Example 2 (rosuvastatin single tablets) showing a release rate similar to that of the Crestor^{®} 10 mg tablet, a control drug, was prepared and its release rate was evaluated. In the formulation of Comparative Examples 4 and 6 affected by a swellable polymer, there occurred a delay in disintegration of the immediate-release granule layer, thus lowering the release rate. This is the same as the result of the release test of atorvastatin evaluated previously, and it was confirmed that fast-release granules are immediately released by the effect of coating of a water-insoluble polymer in preparing combination formulations of sustained-release granules and immediate-release granules.

### Experimental Example 4: Stability test - interaction between atorvastatin calcium salt and excipients

In order to select the most appropriate excipient for the stability of atorvastatin calcium salts, a chemical stability test was performed between the atorvastatin calcium salts and excipients. Specifically, 1 g of atorvastatin calcium salt and 5 g each of the excipients were respectively mixed at room temperature, and packed into vials in a powdered state. The vials were stored for 4 weeks at stress conditions (60°C, 80% relative humidity), the impurity contents (%) were examined via HPLC, and the results are shown in Table 13 below.

The conditions used for HPLC are as follows.
Column: Gemini (C18, 250 mm × 4.6 mm, 5 µm)
Detector: spectrophotometric detector (244 nm)
Mobile phase: 0.05 M ammonium citrate (pH 4.0):ACN:THF = 53:27:20
Flow rate: 1.5 mL/min

**Table 13**

| Sample | Initial Stage | 4 Weeks |
|---|---|---|
| Atorvastatin calcium salt | 0.02 | 0.13 |
| Atorvastatin calcium salt / Eudragit^{®} RS PO | 0.06 | 0.22 |
| Atorvastatin calcium salt / Eudragit^{®} S100 | 0.06 | 0.27 |
| Atorvastatin calcium salt / Kollicoat^{®} SR 30D | 0.06 | 0.29 |
| Atorvastatin calcium salt / cetyl alcohol | 0.05 | 0.22 |
| Atorvastatin calcium salt / Ethocel^{®} Std 14 | 0.05 | 0.23 |
| Atorvastatin calcium salt / Polyox^{®} WSR301 | 0.10 | 0.53 |
| Atorvastatin calcium salt / Metolose^{®} 90SH-100,000cps | 0.08 | 0.37 |
| Atorvastatin calcium salt / metformin hydrochloride | 0.07 | 0.31 |

As can be seen in Table 13, atorvastatin calcium salts showed various values of total impurities depending on the excipients mixed therewith. The values of total impurities of other constituting components of the combination formulation, such as metformin hydrochloride, and swellable polymers, such as polyethylene oxide (Polyox^{®} WSR301) and hypromellose (Metolose^{®} 90SH-100,000cps), showed relatively high total impurity values compared to those of water-insoluble polymers. Accordingly, it was confirmed that when these constituting components are directly brought into contact with atorvastatin calcium salt, it significantly decreases the stability of the combination formulations containing the same.

Among the water-insoluble polymers, the increases in the amount of impurities in the methacrylic acid copolymer (Eudragit^{®} RS PO), waxes (cetyl alcohol), and ethylcellulose (Ethocel^{®} Std 14) were shown to be lower than those when other water-insoluble polymers were used with a swellable polymer and metformin hydrochloride in preparing combination formulations, thus allowing more advantageous formulations to be secured than when the formulations were prepared using the existing methods of preparing suspended-release metformin formulation types known in the art, from the aspect of stability.

From the foregoing, those of ordinary skill in the art will recognize that the present invention may be embodied in other specific forms without departing from its spirit or essential characteristics. The described embodiments are to be considered in all respects only as illustrative and not restrictive. The scope of the present invention is, therefore, indicated by the appended claims rather than by the foregoing description. All changes which come within the meaning and range of equivalency of the claims are to be embraced within the scope of the present invention.

## Claims

1. A combination formulation comprising:
a first sustained-release composition, which comprises granules comprising metformin or a pharmaceutically acceptable salt thereof and a swellable polymer, and a water-insoluble polymer film for coating the granules; and
a second immediate-release composition comprising an HMG-CoA reductase inhibitor.

2. The combination formulation of claim 1, wherein the swellable polymer is at least one selected from the group consisting of hydroxypropyl methylcellulose, hydroxyethylcellulose, hydroxypropylcellulose, polyethylene oxide, carrageenan, natural gum, guar gum, tragacanth, acacia gum, locust bean gum, xanthan gum, polyvinyl alcohol, and polyvinylpyrrolidone.

3. The combination formulation of claim 1 or 2, wherein the swellable polymer has a viscosity of 100 cps or higher.

4. The combination formulation of anyone of claims 1 to 3, wherein the water-insoluble polymer is at least one selected from the group consisting of methacrylic acid copolymer, ethylcellulose, cellulose acetate succinate, cellulose acetate phthalate, fatty acids, fatty acid esters, fatty acid alcohols, and waxes.

5. The combination formulation of claim 4, wherein the fatty acid and fatty acid ester are at least one selected from the group consisting of glyceryl palmitostearate, glyceryl stearate, glyceryl behenate, cetyl palmitate, glyceryl monooleate, stearic acid, and a mixture thereof; the fatty acid alcohol is at least one selected from the group consisting of cetostearyl alcohol, cetyl alcohol, stearyl alcohol, and a mixture thereof; and
the wax is at least one selected from the group consisting of carnauba wax, beeswax, microcrystalline wax, and a mixture thereof.

6. The combination formulation of anyone of claims 1 to 5, wherein metformin or a pharmaceutically acceptable salt thereof is contained in an amount from 250 mg to 1000 mg.

7. The combination formulation of anyone of claims 1 to 6, wherein the water-insoluble polymer is contained in an amount from 1 wt% to 20 wt% based on the total weight of the first sustained-release composition.

8. The combination formulation of anyone of claims 1 to 7, wherein the swellable polymer is contained in an amount from 1 wt% to 40 wt% based on the total weight of the first sustained-release composition.

9. The combination formulation of anyone of claims 1 to 8, wherein the HMG-CoA reductase inhibitor is at least one selected from the group consisting of rosuvastatin, atorvastatin, pitavastatin, lovastatin, simvastatin, pravastatin, and fluvastatin.

10. The combination formulation of anyone of claims 1 to 9, wherein the HMG-CoA reductase inhibitor is contained in an amount from 5 mg to 160 mg.

11. The combination formulation of anyone of claims 1 to 10, wherein the second immediate-release composition is completely disintegrated in distilled water at 37°C within 5 minutes.

12. The combination formulation of anyone of claims 1 to 11, the combination formulation is formulated so that physical contact or chemical reaction between the swellable polymer in the first sustained-release composition and the HMG-CoA reductase inhibitor is inhibited by the water-insoluble polymer film.

13. The combination formulation of anyone of claims 1 to 12, wherein the combination formulation is an uncoated tablet, a film-coated tablet, a double-layer tablet, a multi-layer tablet, or a core tablet and/or
, wherein the first sustained-release composition and the second immediate-release composition are in a form of a double-layer tablet.

14. A method of preparing the combination formulation of anyone of claims 1 to 13, comprising:
preparing the first sustained-release composition by preparing granules comprising metformin or a pharmaceutically acceptable salt thereof, and a swellable polymer, followed by forming a water-insoluble polymer film on the granules;
preparing the second immediate-release composition comprising an HMG-CoA reductase inhibitor; and
formulating the first sustained-release composition and the second immediate-release composition into a unit formulation.

15. The method of claim 14, further comprising forming a film layer on the external surface of the combination formulation.

## Patentansprüche

1. Kombinationsformulierung, umfassend:
eine erste anhaltend freisetzende Zusammensetzung, die ein Granulat, umfassend Metformin oder ein pharmazeutisch akzeptables Salz von diesem und ein quellfähiges Polymer, und einen wasserunlöslichen Polymerfilm zur Beschichtung des Granulats umfasst; und
eine zweite sofort freisetzende Zusammensetzung, umfassend einen HMG-CoA-Reduktasehemmer.

2. Kombinationsformulierung nach Anspruch 1, worin das quellfähige Polymer mindestens eines, ausgewählt aus der Gruppe bestehend aus Hydroxypropylmethylcellulose, Hydroxyethylcellulose, Hydroxypropylcellulose, Polyethylenoxid, Carrageen, Naturgummi, Guargummi, Traganth, Akaziengummi, Johannisbrotkernmehl, Xanthangummi, Polyvinylalkohol und Polyvinylpyrrolidon, ist.

3. Kombinationsformulierung nach Anspruch 1 oder 2, worin das quellfähige Polymer eine Viskosität von 100 cps oder höher aufweist.

4. Kombinationsformulierung nach einem der Ansprüche 1 bis 3, worin das wasserunlösliche Polymer mindestens eines, ausgewählt aus der Gruppe bestehend aus Methacrylsäure-Copolymer, Ethylcellulose, Celluloseacetatsuccinat, Celluloseacetatphthalat, Fettsäuren, Fettsäureestern, Fettsäurealkoholen und Wachsen, ist.

5. Kombinationsformulierung nach Anspruch 4, worin die Fettsäure und der Fettsäureester mindestens eines, ausgewählt aus der Gruppe bestehend aus Glycerylpalmitostearat, Glycerylstearat, Glycerylbehenat, Cetylpalmitat, Glycerylmonooleat, Stearinsäue und einem Gemisch aus diesen, sind;
der Fettsäurealkohol mindestens eines, ausgewählt aus der Gruppe bestehend aus Cetostearylalkohol, Cetylalkohol, Stearylalkohol und einem Gemisch aus diesen, ist; und
das Wachs mindestens eines, ausgewählt aus der Gruppe bestehend aus Carnaubawachs, Bienenwachs, mikrokristallinem Wachs und einem Gemisch aus diesen, ist.

6. Kombinationsformulierung nach einem der Ansprüche 1 bis 5, worin Metformin oder ein pharmazeutisch akzeptables Salz von diesem in einer Menge von 250 mg bis 1000 mg enthalten ist.

7. Kombinationsformulierung nach einem der Ansprüche 1 bis 6, worin das wasserunlösliche Polymer in einer Menge von 1 Gew.-% bis 20 Gew.-%, bezogen auf das Gesamtgewicht der ersten anhaltend freisetzenden Zusammensetzung, enthalten ist.

8. Kombinationsformulierung nach einem der Ansprüche 1 bis 7, worin das quellfähige Polymer in einer Menge von 1 Gew.-% bis 40 Gew.-%, bezogen auf das Gesamtgewicht der ersten anhaltend freisetzenden Zusammensetzung, enthalten ist.

9. Kombinationsformulierung nach einem der Ansprüche 1 bis 8, worin der HMG-CoA-Reduktasehemmer mindestens einer, ausgewählt aus der Gruppe bestehend aus Rosuvastatin, Atorvastatin, Pitavastatin, Lovastatin, Simvastatin, Pravastatin und Fluvastatin, ist.

10. Kombinationsformulierung nach einem der Ansprüche 1 bis 9, worin der HMG-CoA-Reduktasehemmer in einer Menge von 5 mg bis 160 mg enthalten ist.

11. Kombinationsformulierung nach einem der Ansprüche 1 bis 10, worin die zweite sofort freisetzende Zusammensetzung in destilliertem Wasser bei 37°C innerhalb von 5 Minuten vollständig zerfällt.

12. Kombinationsformulierung nach einem der Ansprüche 1 bis 11, wobei die Kombinationsformulierung so formuliert ist, dass ein physischer Kontakt oder eine chemische Reaktion zwischen dem quellfähigen Polymer in der ersten anhaltend freisetzenden Zusammensetzung und dem HMG-CoA-Reduktasehemmer durch den wasserunlöslichen Polymerfilm verhindert wird.

13. Kombinationsformulierung nach einem der Ansprüche 1 bis 12, wobei die Kombinationsformulierung eine unbeschichtete Tablette, eine filmbeschichtete Tablette, eine Doppelschichttablette, eine Mehrschichttablette oder eine Kerntablette ist, und/oder
wobei die erste anhaltend freisetzende Zusammensetzung und die zweite sofort freisetzende Zusammensetzung in der Form einer Doppelschichttablette vorliegen.

14. Verfahren zur Herstellung einer Kombinationsformulierung nach einem der Ansprüche 1 bis 13, umfassend:
Herstellen einer ersten anhaltend freisetzenden Zusammensetzung durch Präparieren eines Granulats, umfassend Metformin oder ein pharmazeutisch akzeptables Salz von diesem und ein quellfähiges Polymer, gefolgt durch Bilden eines wasserunlöslichen Polymerfilms auf dem Granulat;
Herstellen der zweiten sofort freisetzenden Zusammensetzung, umfassend einen HMG-CoA-Reduktasehemmer; und
Formulieren der ersten anhaltend freisetzenden Zusammensetzung und der zweiten sofort freisetzenden Zusammensetzung zu einer Einheiten-Formulierung.

15. Verfahren nach Anspruch 14, außerdem umfassend das Bilden einer Filmschicht auf der äußeren Oberfläche der Kombinationsformulierung.

## Revendications

1. Formulation combinée comprenant :
une première composition à libération prolongée, qui comprend des granules comprenant de la metformine ou l'un de ses sels pharmaceutiquement acceptables et un polymère gonflable, et un film polymère insoluble dans l'eau pour enrober les granules ; et
une seconde composition à libération immédiate comprenant un inhibiteur de la HMG-CoA réductase.

2. Formulation combinée selon la revendication 1, dans laquelle le polymère gonflable est au moins l'un choisi dans le groupe constitué d'hydroxypropylméthyl-cellulose, hydroxyéthylcellulose, hydroxypropylcellulose, oxyde de polyéthylène, carraghénane, gomme naturelle, gomme guar, gomme adragante, gomme arabique, gomme de caroube, gomme xanthane, alcool polyvinylique, et polyvinylpyrrolidone.

3. Formulation combinée selon la revendication 1 ou 2, dans laquelle le polymère gonflable présente une viscosité de 100 cps ou supérieure.

4. Formulation combinée selon l'une quelconque des revendications 1 à 3, dans laquelle le polymère insoluble dans l'eau est au moins l'un choisi dans le groupe constitué de copolymère d'acide méthacrylique, éthylcellulose, acétosuccinate de cellulose, acétophtalate de cellulose, acides gras, esters d'acides gras, alcools d'acides gras, et cires.

5. Formulation combinée selon la revendication 4, dans laquelle l'acide gras et l'ester d'acide gras sont au moins l'un choisi dans le groupe constitué de palmitostéarate de glycéryle, stéarate de glycéryle, béhénate de glycéryle, palmitate de cétyle, monooléate de glycéryle, acide stéarique, et l'un de leurs mélanges ;
l'alcool d'acides gras est au moins l'un choisi dans le groupe constitué de l'alcool cétostéarylique, l'alcool cétylique, l'alcool stéarylique, et l'un de leurs mélanges ; et
la cire est au moins l'une choisie dans le groupe constitué de cire de carnauba, cire d'abeille, cire microcristalline, et l'un de leurs mélanges.

6. Formulation combinée selon l'une quelconque des revendications 1 à 5, dans laquelle la metformine ou l'un de ses sels pharmaceutiquement acceptables est contenue en une quantité de 250 mg à 1000 mg.

7. Formulation combinée selon l'une quelconque des revendications 1 à 6, dans laquelle le polymère insoluble dans l'eau est contenu en une quantité de 1 % en poids à 20 % en poids sur la base du poids total de la première composition à libération prolongée.

8. Formulation combinée selon l'une quelconque des revendications 1 à 7, dans laquelle le polymère gonflable est contenu en une quantité de 1 % en poids à 40 % en poids sur la base du poids total de la première composition à libération prolongée.

9. Formulation combinée selon l'une quelconque des revendications 1 à 8, dans laquelle l'inhibiteur de la HMG-CoA réductase est au moins l'un choisi dans le groupe constitué de rosuvastatine, atorvastatine, pitavastatine, lovastatine, simvastatine, pravastatine, et fluvastatine.

10. Formulation combinée selon l'une quelconque des revendications 1 à 9, dans laquelle l'inhibiteur de la HMG-CoA réductase est contenu en une quantité de 5 mg à 160 mg.

11. Formulation combinée selon l'une quelconque des revendications 1 à 10, dans laquelle la seconde composition à libération immédiate est complètement désintégrée dans de l'eau distillée à 37 °C en 5 minutes.

12. Formulation combinée selon l'une quelconque des revendications 1 à 11, la formulation combinée étant formulée de telle façon que le contact physique ou la réaction chimique entre le polymère gonflable dans la première composition à libération prolongée et l'inhibiteur de la HMG-CoA réductase est inhibé par le film polymère insoluble dans l'eau.

13. Formulation combinée selon l'une quelconque des revendications 1 à 12, dans laquelle la formulation combinée est un comprimé non enrobé, un comprimé enrobé d'un film, un comprimé à double couche, un comprimé à couches multiples, ou un noyau de comprimé et/ou
dans laquelle la première composition à libération prolongée et la seconde composition à libération immédiate sont sous une forme de comprimé à double couche.

14. Procédé de préparation de la formulation combinée selon l'une quelconque des revendications 1 à 13, comprenant :
la préparation de la première composition à libération prolongée en préparant les granules comprenant la metformine ou l'un de ses sels pharmaceutiquement acceptables, et un polymère gonflable, suivie de la formation d'un film polymère insoluble dans l'eau sur les granules ;
la préparation de la seconde composition à libération immédiate comprenant un inhibiteur de la HMG-CoA réductase ; et
la formulation de la première composition libération prolongée et de la seconde composition à libération immédiate dans une formulation unitaire.

15. Procédé selon la revendication 14, comprenant en outre la formation d'une couche de film sur la surface externe de la formulation combinée.
